# EUROPÄISCHE PATENTSCHRIFT

(11) **EP 3 207 893 B1**
(45) Veröffentlichungstag und Bekanntmachung des Hinweises auf die Patenterteilung: **17.04.2024**
(21) Anmeldenummer: 17156567.4
(22) Anmeldetag: 16.02.2017
(51) Int. Cl.: A61B 18/12, A61B 18/00, H03K 17/955, H03K 17/96

(54) **CHIRURGIEVORRICHTUNG MIT FUNKTIONSVORRICHTUNG**
SURGICAL INSTRUMENT WITH FUNCTIONAL DEVICE
DISPOSITIF CHIRURGICAL COMPRENANT UN DISPOSITIF FONCTIONNEL

(30) Priorität: 17.02.2016 DE 102016202456
(43) Veröffentlichungstag der Anmeldung: 23.08.2017
(73) Patentinhaber: Olympus Winter & Ibe GmbH, 22045 Hamburg (DE)
(72) Erfinder: Avcioglu, Erdinc, 22589 Hamburg (DE)
(74) Vertreter: Eisenführ Speiser

(56) Entgegenhaltungen:
- EP-A1- 0 017 318
- WO-A1-2011/080308
- CN-U- 204 707 112
- DE-A1- 2 712 734
- US-A- 5 108 389
- US-A1- 2011 169 506

## Beschreibung

Die Erfindung betrifft eine Chirurgievorrichtung mit einem Chirurgiegenerator, einem Schalter, einer Funktionsvorrichtung, insbesondere Rauchgasabsaugvorrichtung und einem kapazitiven Sensor. Weiterhin betrifft die Erfindung ein Verfahren zum automatischen Aktivieren und Deaktivieren einer Funktionsvorrichtung, insbesondere Rauchgasabsaugvorrichtung einer Chirurgievorrichtung.

Aus EP 0 017 318 A1 ist ein kapazitiver Schaltkreis zum Betreiben einer ferngesteuerten Vorrichtung bekannt. Aus DE 27 12 734 A1 ist eine Anordnung zur Steuerung der elektrischen Leistung zahnärztlicher Handinstrumente bekannt.

Chirurgievorrichtungen mit Funktionsvorrichtungen, wie beispielsweise Rauchgasabsaugvorrichtungen sind zum Beispiel aus der US 5,108,389 A bekannt. Chirurgievorrichtungen können eine oder mehrere Funktionsvorrichtungen aufweisen, die beim Aktivieren bestimmte Funktionen, wie beispielsweise das Absaugen von Rauchgas übernehmen. Solche Chirurgievorrichtungen werden typischerweise in Operations-Sälen, OPs oder Behandlungszimmern bei der Behandlung von Patienten verwendet. Beim Schneiden und Koagulieren von Gewebe mit energiebasierten Chirurgieinstrumenten, beispielsweise Hochfrequenz-Chirurgieinstrumenten, wird Gewebe verdampft und Körperflüssigkeit vaporisiert, wodurch ein Rauchgas entsteht, das verdampftes Gewebe und vaporisierte Körperflüssigkeit in Form von Rauchgaspartikeln enthält. Dieses Rauchgas kann die Sicht auf das zu behandelnde Gewebe erschweren und gesundheitliche Risiken bergen. Daher werden Rauchgasabsaugvorrichtungen verwendet, um das Rauchgas aus dem OP zu entfernen. Eine andere Funktionsvorrichtung kann beispielsweise eine Beleuchtungsvorrichtung, wie zum Beispiel eine Lampe sein, die bei Aktivierung die Sicht auf das zu behandelnde Gewebe verbessern kann.

Bei chirurgischer Rauchgasabsaugung werden unterschiedliche Verfahren und Vorrichtungen verwendet, um die Rauchgasabsaugvorrichtung nur dann zu aktivieren, wenn sie auch benötigt wird. Die Rauchgasabsaugvorrichtung kann manuell oder automatisch aktiviert werden. Für das manuelle Aktivieren, kann die Chirurgievorrichtung einen Schalter aufweisen, der beispielsweise am Chirurgieinstrument angeordnet sein kann oder als Fußschalter mit dem Chirurgieinstrument verbunden sein kann. Beim manuellen Aktivieren wird die Rauchgasabsaugvorrichtung entweder durch manuelles Betätigen des Schalters am Chirurgieinstrument oder durch manuelles Betätigen des Fußschalters aktiviert. Beim automatischen Aktivieren werden verschiedene Vorrichtungen und Verfahren verwendet, um den Chirurgiegenerator, beispielsweise einen Elektrochirurgiegenerator und die Rauchgasabsaugvorrichtung synchronisiert zu aktivieren.

Bei einer ersten bekannten Chirurgievorrichtung wird zum automatischen Aktivieren der Rauchgasabsaugvorrichtung ein Stromfluss durch ein Kabel, das den Chirurgiegenerator mit dem Chirurgieinstrument verbindet, induktiv detektiert. Der Stromfluss kann beispielsweise direkt im Chirurgieinstrument oder durch eine am Kabel angeordnete Detektionsvorrichtung detektiert werden. In Reaktion auf einen detektierten Stromfluss durch das Kabel kann vom Chirurgieinstrument oder von der Detektionsvorrichtung ein Signal an die Rauchgasabsaugvorrichtung gesendet werden, um diese zu aktivieren. Wenn kein Stromfluss mehr detektiert wird, kann ein Signal zum Deaktivieren der Rauchgasabsaugvorrichtung gesendet werden.

Bei einer zweiten bekannten Chirurgievorrichtung wird ausgenutzt, dass Chirurgiegeneratoren typischerweise voneinander unterscheidbare akustische Signale, beispielsweise Töne, bei der Aktivierung der sogenannten "cut" und sogenannten "coagulation"-Funktion abgeben. Diese Chirurgievorrichtung weist einen akustischen Sensor auf, der die Rauchgasabsaugvorrichtung dann aktiviert, wenn ein vorbestimmter Ton detektiert wird. Das Deaktivieren der Rauchgasabsaugvorrichtung kann erfolgen, wenn der akustische Sensor einen entsprechenden vom Chirurgiegenerator ausgegebenen Ton detektiert.

Bei einer dritten bekannten Chirurgievorrichtung ist der Chirurgiegenerator direkt mit der Rauchgasabsaugvorrichtung verbunden, beispielsweise indem der Netzstecker des Chirurgiegenerators direkt an der Rauchgasabsaugvorrichtung angeschlossen ist. Die Rauchgasabsaugvorrichtung kann die Stromaufnahme des Chirurgiegenerators messen und bei entsprechender Kalibrierung kann die Aktivierung der Rauchgasabsaugvorrichtung mit der Aktivierung des Chirurgiegenerators synchronisiert sein. In diesem Fall kann die Rauchgasabsaugvorrichtung automatisch aktiviert werden, wenn der Chirurgiegenerator aktiviert wird.

Ziel der Erfindung ist es, eine verbesserte Chirurgievorrichtung mit einer Funktionsvorrichtung bereitzustellen.

Dieses Ziel wird erreicht durch eine Chirurgievorrichtung zum im Wesentlichen synchronen automatischen Aktivieren und Deaktivieren einer Funktionsvorrichtung und eines Chirurgiegenerators der Chirurgievorrichtung. Die Chirurgievorrichtung umfasst den Chirurgiegenerator, die Funktionsvorrichtung, einen Schalter und einen kapazitiven Sensor. Der Chirurgiegenerator ist ausgebildet Energie für ein energiebasiertes Chirurgieinstrument bereitzustellen. Die Funktionsvorrichtung ist ausgebildet in einem aktivierten Zustand eine Funktion bereitzustellen. Der Schalter dient zum Aktivieren und Deaktivieren des Chirurgiegenerators. Der kapazitive Sensor weist wenigstens eine Messelektrode auf. Die Messelektrode ist an dem Schalter angeordnet. Der kapazitive Sensor ist ausgebildet eine Kapazitätsänderung oder Kapazität an der Messelektrode zu messen und in Abhängigkeit von der gemessenen Kapazitätsänderung oder Kapazität die Funktionsvorrichtung zu aktivieren oder zu deaktivieren.

Bevorzugt ist die Funktionsvorrichtung eine Rauchgasabsaugvorrichtung, die ausgebildet ist in einem aktivierten Zustand Rauchgas abzusaugen. Die Funktionsvorrichtung kann auch eine Beleuchtungsvorrichtung sein, die ausgebildet ist in einem aktivierten Zustand ein spezielles Licht bereitzustellen, das es ermöglicht die Sicht zu verbessern. Ferner kann die Funktionsvorrichtung ein Blinklicht aufweisen, das dazu ausgebildet ist dem Nutzer der Chirurgievorrichtung anzuzeigen, dass die Funktionsvorrichtung aktiviert wurde und/oder dass bei weiterer Betätigung des Schalters der Chirurgiegenerator aktiviert wird. Das Blinklicht kann beispielsweise eine Warnungs-Funktion erfüllen. Des Weiteren kann die Funktionsvorrichtung eine Steuerung für ein Ladegerät sein oder diese aufweisen, die dazu ausgebildet ist in einem aktivierten Zustand eine Energiequelle, beispielsweise des energiebasierten Chirurgieinstruments zu laden. Weiterhin kann die Funktionsvorrichtung eine Videoaufzeichnungsvorrichtung zur Aufzeichnung von Videodaten oder Fotodaten sein oder eine Videoaufzeichnungsvorrichtung aufweisen. Des Weiteren kann die Funktionsvorrichtung eine Audioaufzeichnungsvorrichtung zur Aufzeichnung von Audiodaten, insbesondere Sprachdaten sein oder eine Audioaufzeichnungsvorrichtung aufweisen. Ferner kann die Funktionsvorrichtung eine Temperaturregulierungsvorrichtung sein, die dazu ausgebildet ist ein energiebasiertes Elektrochirurgieinstrument und/oder ein Endoskop zu erwärmen und/oder zu kühlen. Die Funktionsvorrichtung kann auch eine solche Temperaturregulierungsvorrichtung aufweisen. Die Funktionsvorrichtung kann des Weiteren eine Steuerungseinheit sein, die dazu ausgebildet ist weitere externe Vorrichtungen, wie beispielsweise einen Computer, Mobiltelefon, Tablet-Computer oder dergleichen in einem aktivierten Zustand zu steuern. Die Steuerungseinheit kann beispielsweise auch dazu ausgebildet sein eine Pumpe, beispielsweise zur Kühlmittelversorgung zu steuern. Es können auch mehrere Funktionsvorrichtungen mit Hilfe des kapazitiven Sensors aktiviert werden.

Wenn die Rauchgasabsaugvorrichtung aktiviert ist, saugt sie Gas ein und dient insbesondere dazu Rauchgas abzusaugen. Wenn der Chirurgiegenerator aktiviert ist, stellt er Energie bereit, die insbesondere für das Betreiben eines energiebasierten Chirurgieinstruments verwendet werden kann.

Der kapazitive Sensor kann die Kapazitätsänderung an der Messelektrode, die Kapazität an der Messelektrode oder Kapazitätsänderung und Kapazität an der Messelektrode messen und davon abhängig die Funktionsvorrichtung aktivieren oder deaktivieren. Der kapazitive Sensor kann beispielsweise ein Berührungssensor, Annäherungssensor oder ähnlicher kapazitiver Sensor sein oder einen oder mehrere solcher Sensoren aufweisen. Die Kapazität an der Messelektrode ist davon abhängig, wie weit eine Fläche von der Messelektrode entfernt ist oder ob eine Fläche auf der Messelektrode platziert ist. Eine Fläche kann beispielsweise eine Oberfläche eines Objektes, wie die Oberfläche eines Fingers für einen Handschalter oder die Oberfläche eines Fußes für einen Fußschalter, sein. Die Oberfläche des Fußes und die Oberfläche der Messelektrode bilden im Wesentlichen zwei sich gegenüberliegende Oberflächen eines Kondensators. Die Kapazität dieses Kondensators hängt von verschiedenen Faktoren, insbesondere dem Abstand der Oberflächen ab. Der kapazitive Sensor kann derart eingestellt sein, dass dieser ein Aktivierungssignal zum Aktivieren oder ein Deaktivierungssignal zum Deaktivieren an die Funktionsvorrichtung übermittelt, wenn zum Beispiel ein vorbestimmter Schwellenwert der Kapazität und/oder der Kapazitätsänderung überschritten oder unterschritten wird.

Der kapazitive Sensor weist außerdem eine Steuerlogik auf, die aus einem mittels der Messelektrode gewonnenen Messwert ein Steuersignal zum Aktivieren oder Deaktivieren der Funktionsvorrichtung generiert.

Die Chirurgievorrichtung ermöglicht ein im Wesentlichen synchrones Aktivieren des Chirurgiegenerators und der Funktionsvorrichtung. Bevorzugt erfolgt das Aktivieren der Funktionsvorrichtung bei oder kurz vor dem Aktivieren des Chirurgiegenerators. Hierfür kann beispielsweise ein vorbestimmter Schwellenwert für die Kapazität und/oder Kapazitätsänderung derart definiert sein, dass der kapazitive Sensor die Funktionsvorrichtung aktiviert, wenn der Schalter betätigt wird, auf dem die Messelektrode des kapazitiven Sensors angeordnet ist. Hierdurch wird zuerst die Funktionsvorrichtung und unmittelbar anschließend oder zeitgleich bzw. synchron der Chirurgiegenerator aktiviert. Der Chirurgiegenerator kann mit einem energiebasierten Chirurgieinstrument, beispielsweise einem Ultraschallchirurgieinstrument, einem Laserchirurgieinstrument, einem Elektrochirurgieinstrument oder ähnlichem energiebasierten Chirurgieinstrument verbunden sein. Dieses wird von dem Chirurgiegenerator mit Energie versorgt, so dass mit diesem Gewebe geschnitten und/oder koaguliert werden kann. Durch die Verwendung des energiebasierten Chirurgieinstruments zur Behandlung von Gewebe entsteht Rauchgas. Dieses Rauchgas kann beispielsweise von einer Funktionsvorrichtung in Form einer Rauchgasabsaugvorrichtung abgesaugt werden.

Die Rauchgasabsaugvorrichtung kann beispielsweise eine Pumpe aufweisen, die über ein Lumen einer Leitung, wie beispielsweise eines Schlauches mit dem energiebasierten Chirurgieinstrument verbunden ist. Das energiebasierte Chirurgieinstrument kann eine Eintrittsöffnung aufweisen, die bevorzugt nahe dem Schneid- und/oder Koagulationswerkzeug des energiebasierten Chirurgieinstruments angeordnet ist und mit dem Lumen der Leitung verbunden ist. Über die Eintrittsöffnung und das Lumen der Leitung kann das Rauchgas abgesaugt werden, wodurch eine effiziente Entfernung des Rauchgases in unmittelbarer Nähe zu dessen Entstehungsort möglich ist.

Die Erfindung schließt die Erkenntnis ein, dass die bekannten Vorrichtungen und Verfahren zum Aktivieren und Deaktivieren des Chirurgiegenerators und einer Funktionsvorrichtung diverse Nachteile haben. Die Erfindung hat diese Nachteile nicht oder kann diese Nachteile zumindest verringern. Gegenüber der manuellen Aktivierung ermöglicht die Erfindung auf zusätzliche Handlungen, die beim manuellen Aktivieren des Chirurgiegenerator und der Funktionsvorrichtung notwendig sind, zu verzichten. Beispielsweise muss bei der manuellen Aktivierung in einem ersten Schritt die Funktionsvorrichtung manuell aktiviert werden und in einem zweiten Schritt der Chirurgiegenerator manuell aktiviert werden. Die Erfindung ermöglicht ein nahezu synchrones Aktivieren der Funktionsvorrichtung und des Chirurgiegenerators ohne zusätzlichen Verfahrensschritt. Dies verbessert die Bedienfreundlichkeit der Chirurgievorrichtung. Das automatische Aktivieren mittels der ersten bekannten Chirurgievorrichtung, die eine Funktionsvorrichtung in Form einer Rauchgasabsaugvorrichtung aktiviert, wenn ein Stromfluss durch das Kabel zwischen energiebasierten Chirurgieinstrument und Chirurgiegenerator induktiv detektiert wird, funktioniert bei abgeschirmten Kabeln nicht verlässlich, so dass eine automatische Aktivierung der Funktionsvorrichtung in der Hälfte der Fälle scheitert. Demgegenüber ermöglicht die Erfindung auch eine Verwendung von abgeschirmten Kabeln. Das automatische Aktivieren mit Hilfe der zweiten bekannten Chirurgievorrichtung, die akustische Signale, wie Töne mit einem akustischen Sensor detektiert, um in Reaktion auf die Detektion eine Funktionsvorrichtung in Form einer Rauchgasabsaugvorrichtung zu aktivieren, setzt voraus, dass die akustischen Signale, beispielsweise Töne unterschiedlicher Chirurgiegeneratoren gleich sind. Dies ist für unterschiedliche Chirurgiegeneratoren typischerweise nicht der Fall. Daher können nur auf den akustischen Sensor abgestimmte Chirurgiegeneratoren in der zweiten bekannten Chirurgievorrichtung verwendet werden. Ferner besteht in diesem Fall das Problem, dass Störgeräusche die Detektion des akustischen Signals behindern können. Die dritte bekannte Chirurgievorrichtung, bei der der Netzstecker des Chirurgiegenerators direkt an eine Funktionsvorrichtung in Form einer Rauchgasabsaugvorrichtung angeschlossen ist, setzt voraus, dass der Chirurgiegenerator einen nach IEC320 genormten Kaltgerätestecker hat, um kompatibel zu sein. Ferner muss der Chirurgiegenerator in einem gewissen Leistungsbereich arbeiten, damit dieser mit der Funktionsvorrichtung kompatibel ist. Daher können nicht alle Chirurgiegeneratoren in der dritten bekannten Chirurgievorrichtung verwendet werden. Insbesondere wenn die Chirurgievorrichtung mehrere Funktionsvorrichtungen aufweist, kann es sich schwierig gestalten diese untereinander kompatibel zu halten. Alle bekannten Chirurgievorrichtungen und Verfahren zum Aktivieren der Funktionsvorrichtung bedürfen einer Kompatibilität seitens des Chirurgiegenerators mit der Funktionsvorrichtung.

Ein Aspekt der Erfindung ist es, dass keine Kompatibilität seitens des Chirurgiegenerators mit der Funktionsvorrichtung notwendig ist, da der Chirurgiegenerator vom Schalter aktiviert und deaktiviert werden kann und die Funktionsvorrichtung vom kapazitiven Sensor aktiviert und deaktiviert werden kann. Der Chirurgiegenerator und die Funktionsvorrichtung können daher in unterschiedlichen Leistungsbereichen arbeiten und müssen nicht zueinander kalibriert sein. Ferner müssen auch keine Kompatibilitätsbedingungen seitens des Chirurgiegenerators, des energiebasierten Chirurgieinstruments und des Schalters erfüllt werden, um diese mit der Funktionsvorrichtung in der Chirurgievorrichtung zu koppeln und zu verwenden. Das nahezu synchrone Aktivieren der Funktionsvorrichtung, wenn der Chirurgiegenerator aktiviert wird, ermöglicht es den Energieverbrauch zu verringern, da die Funktionsvorrichtung nicht dauerhaft betrieben werden muss. Des Weiteren ermöglicht die synchrone automatische Aktivierung einer Funktionsvorrichtung in Form einer Rauchgasabsaugvorrichtung, effektiv Rauchgas abzusaugen. Ferner kann der Geräuschpegel der Chirurgievorrichtung verringert werden, da die Rauchgasabsaugvorrichtung im Wesentlichen nur dann aktiv zu sein braucht, wenn auch Rauchgas erzeugt wird, d.h., wenn der Chirurgiegenerator aktiviert ist und ein energiebasiertes Chirurgieinstrument mit Energie versorgt. Auch die Gefahr eines Festsaugens der Öffnung der Leitung der Rauchgasabsaugvorrichtung an einem Körper kann verringert werden. Weiterhin kann der Schalter autoklavierbar sein und daher im Sterilfeld angeordnet werden.

Der Chirurgiegenerator und/oder die Funktionsvorrichtung können ausgebildet sein, initial eingeschaltet zu werden, um in einen betriebsbereiten Zustand ("standby"-Modus) versetzt zu werden. Das Aktivieren und Deaktivieren der Funktionsvorrichtung erfolgt in diesem Fall in deren betriebsbereitem Zustand. Dies ermöglicht es eine schnellere Reaktionszeit zu erreichen und den Energieverbrauch zu verringern.

In einer bevorzugten Ausgestaltung der Chirurgievorrichtung ist die Messelektrode derart an dem Schalter angeordnet und der kapazitive Sensor derart eingestellt, dass ein Aktivieren des Chirurgiegenerators auch ein Aktivieren der Funktionsvorrichtung bewirkt. Bevorzugt ist die Messelektrode auf dem Schalter aufgeklebt, so dass Betätigen des Schalters auch zu einer Berührung des kapazitiven Sensors, beispielsweise eines Berührungssensors, führt. Dies stellt sicher, dass die Funktionsvorrichtung immer dann aktiviert wird, wenn der Chirurgiegenerator aktiviert wird.

Die Messelektrode ist bevorzugt zusätzlich oder alternativ derart an dem Schalter angeordnet und der kapazitive Sensor ist bevorzugt zusätzlich oder alternativ derart eingestellt, dass ein Deaktivieren der Funktionsvorrichtung auch ein Deaktivieren des Chirurgiegenerators bewirkt. Im Falle einer Funktionsvorrichtung in Form einer Rauchgasabsaugvorrichtung stellt dies sicher, dass beim Deaktivieren der Rauchgasabsaugvorrichtung auch der Chirurgiegenerator deaktiviert wird. Hierdurch wird verhindert, dass Rauchgas von der Chirurgievorrichtung erzeugt wird, während die Rauchgasabsaugvorrichtung deaktiviert ist.

In einer Ausgestaltung können mehrere Messelektroden, beispielsweise zwei Messelektroden des kapazitiven Sensors an dem Schalter angeordnet sein und der kapazitive Sensor derart ausgebildet sein, dass dieser die Funktionsvorrichtung nur aktiviert, wenn an den zwei Messelektroden eine Kapazitätsänderung und/oder Kapazität gemessen wird, die über einem vorbestimmten Schwellenwert liegt. In dieser Ausgestaltung kann der Chirurgiegenerator aktiviert werden, ohne dass auch die Funktionsvorrichtung aktiviert wird, wenn beispielsweise der Schwellenwert der Kapazitätsänderung und/oder Kapazität nur für eine der zwei Messelektroden überschritten wird. Dies wäre beispielsweise bei einem Fußschalter mit je einer Messelektrode pro Fuß der Fall, wenn nur ein Fuß auf eine Messelektrode platziert wird.

In einer besonders bevorzugten Ausgestaltung der Chirurgievorrichtung ist der kapazitive Sensor derart ausgebildet, dass ein Aktivieren oder Deaktivieren der Funktionsvorrichtung im Wesentlichen synchron zum Aktivieren oder Deaktivieren des Chirurgiegenerators erfolgt. Der kapazitive Sensor ist dafür bevorzugt derart auf dem Schalter angeordnet, dass beim oder unmittelbar vor dem Betätigen des Schalters zum Aktivieren des Chirurgiegenerators die Funktionsvorrichtung aktiviert wird und beim oder unmittelbar nach dem Deaktivieren des Chirurgiegenerators auch die Funktionsvorrichtung deaktiviert wird.

Die Chirurgievorrichtung weist bevorzugt eine auf dem Schalter befestigbare kapazitive Messeinrichtung auf. Die kapazitive Messeinrichtung ist bevorzugt eine Folie. Alternativ oder zusätzlich kann die kapazitive Messeinrichtung eine oder mehrere Folien und/oder Drähte aufweisen. Besonders bevorzugt weist die kapazitive Messeinrichtung die Messelektrode des kapazitiven Sensors auf. Die Chirurgievorrichtung kann auch mehrere auf dem Schalter befestigbare kapazitive Messeinrichtungen, beispielsweise Folien aufweisen. Diese können eine oder mehrere Messelektroden des kapazitiven Sensors aufweisen. Bevorzugt ist die kapazitive Messeinrichtung oder sind die kapazitiven Messeinrichtungen auf dem Schalter befestigt. Besonders bevorzugt ist die kapazitive Messeinrichtung oder sind die kapazitiven Messeinrichtungen auf dem Schalter derart aufgeklebt, dass sie sich von diesem im Wesentlichen ohne Rückstände wieder entfernen lässt oder wieder entfernen lassen. Dies ermöglicht es die kapazitive Messeinrichtung, insbesondere in Form einer Folie nach einmaligem Gebrauch zu ersetzen, so dass Einweg-Folien verwendet werden können. Die Verwendung von Einweg-Folien ermöglicht es die Keimbelastung der Chirurgievorrichtung zu verringern. Insbesondere ist der Schalter der Chirurgievorrichtung bevorzugt autoklavierbar, so dass ein Entfernen der Folie zum autoklavieren des Schalters, eine weitestgehende Befreiung von Keimen ermöglicht. Nach dem Autoklavieren wird in diesem Fall vor einer weiteren Verwendung der Chirurgievorrichtung eine neue kapazitive Messeinrichtung, insbesondere in Form einer Folie auf den Schalter befestigt, beispielsweise aufgeklebt.

Die Messelektrode ist bevorzugt als eine Metalloxid-Beschichtung auf die kapazitive Messeinrichtung aufgebracht. Alternativ kann die Messelektrode auch auf der kapazitiven Messeinrichtung aufgebracht sein. Beispielsweise kann die Messelektrode auf der kapazitiven Messeinrichtung aufgeklebt sein.

In einer bevorzugten Ausgestaltung weist der kapazitive Sensor eine Messelektronik auf, die beispielsweise die Steuerlogik des kapazitiven Sensors bilden oder mit dieser verbunden sein kann. Die Messelektronik ist bevorzugt dazu ausgebildet, die Kapazitätsänderung oder die Kapazität an der Messelektrode zu messen. Die Messelektronik des kapazitiven Sensors kann in der Funktionsvorrichtung angeordnet sein. Bevorzugt ist die Messelektrode des kapazitiven Sensors mit der Messelektrode über ein Kabel verbunden. Die Messelektrode und das Kabel sind in diesem Fall bevorzugt einmalig verwendbar. Alternativ kann der kapazitive Sensor auch über ein Kabel mit der Funktionsvorrichtung verbunden sein. In diesem Fall ist die Messelektronik des kapazitiven Sensors an dem Kabel zwischen der Messelektrode und der Funktionsvorrichtung angeordnet. Bevorzugt weist in dieser Ausgestaltung das Kabel ein Messelektronikgehäuse auf. Das Messelektronikgehäuse dient dazu die Messelektronik aufzunehmen. Bevorzugt ist die Messelektrode einmalig verwendbar und das Kabel mit der im Messelektronikgehäuse angeordneten Messelektronik wiederverwendbar. Die Messelektronik kann in diesem Fall Nahe der Messelektrode angeordnet werden. Ein kürzerer Abstand zwischen Messelektrode und Messelektronik ermöglicht eine Verringerung der Störanfälligkeit und Störeinflüsse.

In einer weiteren Ausgestaltung weist der kapazitive Sensor wenigstens eine Schirmelektrode auf. Die Schirmelektrode ist bevorzugt nahe der Messelektrode angeordnet und dazu ausgebildet einen inhomogenen Randbereich des elektrischen Feldes von der Messelektrode abzuschirmen. Dies ermöglicht ein homogeneres elektrisches Messfeld. Die Schirmelektrode kann beispielsweise um die Messelektrode herum angeordnet sein.

In einer besonders bevorzugten Ausgestaltung ist der der Schalter ein Fußschalter. Der Fußschalter ist ausgebildet mit Hilfe eines Fußes oder mehrerer Füße betätigt zu werden und kann beispielsweise ein Fußpedal oder dergleichen sein. Der Fußschalter kann auch mehrere, beispielsweise zwei Fußpedale aufweisen. In diesem Fall kann der Schalter derart ausgebildet sein, dass der Chirurgiegenerator nur dann aktiviert wird, wenn beide Fußpedale zur gleichen Zeit betätigt werden. Der Schalter kann auch derart ausgebildet sein, dass der Chirurgiegenerator dann aktiviert wird, wenn eine der beiden Fußpedale betätigt wird.

Besonders bevorzugt weist die Chirurgievorrichtung ein energiebasiertes Chirurgieinstrument auf. Das energiebasierte Chirurgieinstrument ist bevorzugt dazu ausgebildet Gewebe zu schneiden und/oder zu koagulieren. Das energiebasierte Chirurgieinstrument kann beispielsweise ein Ultraschallchirurgieinstrument, ein Laserchirurgieinstrument, ein Elektrochirurgieinstrument oder anderes energiebasiertes Chirurgieinstrument sein. Das Elektrochirurgieinstrument weist wenigstens eine Elektrode auf und ist dazu ausgebildet mittels einer hochfrequenten Wechselspannung betrieben zu werden, um Gewebe zu schneiden und/oder zu koagulieren. Durch das Schneiden von Gewebe und auch durch Koagulieren von Gewebe kommt es zur Entwicklung von Rauchgas. Dieses Rauchgas kann beispielsweise mit Hilfe einer Funktionsvorrichtung in Form einer Rauchgasabsaugvorrichtung abgesaugt werden. Hierdurch kann die Sicht auf das zu behandelnde Gewebe verbessert werden. Ferner kann Rauchgas auch ein Gesundheitsrisiko sowohl für einen mit der Chirurgievorrichtung Behandelten, als auch für den Verwender der Chirurgievorrichtung darstellen. Die Entfernung des Rauchgases mit Hilfe der Rauchgasabsaugvorrichtung ermöglicht es daher auch das Gesundheitsrisiko des Verwenders und des Behandelten zu verringern. Dadurch, dass die Rauchgasabsaugvorrichtung aktiviert ist, wenn der Chirurgiegenerator aktiviert wird, kann sichergestellt werden, dass die Gesundheitsbelastung möglichst gering ausfällt.

Die kapazitive Messeinrichtung kann einmalig verwendbar sein oder wiederverwendbar. Die kapazitive Messeinrichtung ist derart ausgebildet, dass sie in bekannten Chirurgievorrichtungen verwendet werden kann, d.h. die Chirurgievorrichtungen können mit der kapazitiven Messeinrichtung nachgerüstet werden, um ein synchrones automatisches Aktivieren der Funktionsvorrichtung, beispielsweise der Rauchgasabsaugvorrichtung und des Chirurgiegenerators zu ermöglichen. Die kapazitive Messeinrichtung ist bevorzugt eine Folie. Alternativ oder zusätzlich kann die kapazitive Messeinrichtung eine Folie und/oder einen oder mehrere Drähte aufweisen.

Des Weiteren betrifft die Erfindung ein Verfahren gemäß Anspruch 12.

Bevorzugt ist der Schwellenwert der Kapazitätsänderung oder Kapazität derart gewählt, dass die Funktionsvorrichtung aktiviert wird, wenn der Schalter betätigt wird und deaktiviert wird, wenn das Objekt vom kapazitiven Sensor entfernt wird. Das Objekt kann beispielsweise eine Hand, ein Finger, ein Fuß oder ein anderes Interaktionsobjekt eines Nutzers sein. Durch einen derart gewählten Schwellenwert der Kapazitätsänderung oder Kapazität wird sichergestellt, dass das Aktivieren der Funktionsvorrichtung kurz vor dem Aktivieren des Chirurgiegenerators erfolgt.

Die Erfindung soll nun anhand von in den Figuren schematisch abgebildeten Ausführungsbeispielen näher erläutert werden. Von den Figuren zeigt:
- Fig. 1: eine schematische Darstellung eines ersten Ausführungsbeispiels einer Chirurgievorrichtung;
- Fig. 2: eine schematische Darstellung eines zweiten Ausführungsbeispiels einer Chirurgievorrichtung;
- Fig. 3: eine schematische Darstellung eines dritten Ausführungsbeispiels einer Chirurgievorrichtung;
- Fig. 4: eine schematische Darstellung eines vierten Ausführungsbeispiels einer Chirurgievorrichtung;
- Fig. 5: eine schematische Darstellung eines fünften Ausführungsbeispiels einer Chirurgievorrichtung;
- Fig. 6: ein Blockdiagramm eines Ausführungsbeispiels eines Verfahrens zum im Wesentlichen synchronen automatischen Aktivieren und Deaktivieren einer Funktionsvorrichtung in Form einer Rauchgasabsaugvorrichtung und eines Chirurgiegenerators einer Chirurgievorrichtung.

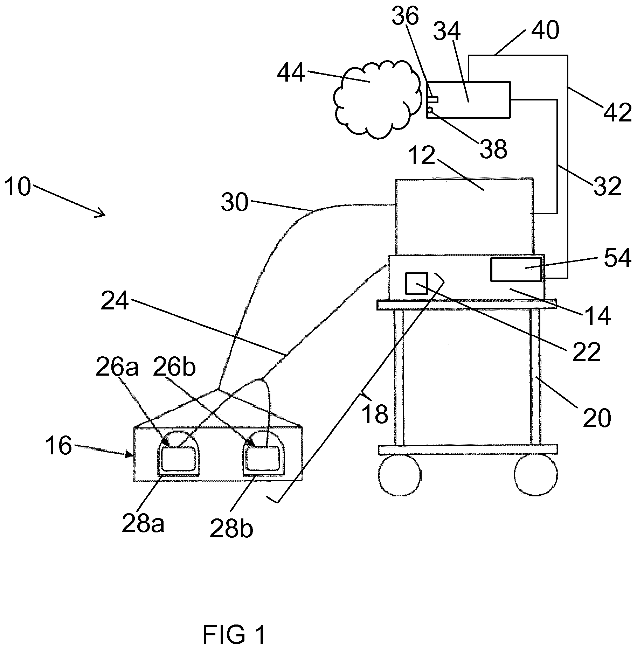

Figur 1 zeigt ein erstes Ausführungsbeispiel einer Chirurgievorrichtung 10. Die Chirurgievorrichtung 10 hat einen Chirurgiegenerator 12, eine Rauchgasabsaugvorrichtung 14, einen Fußschalter 16 und einen kapazitiven Sensor 18. In diesem Ausführungsbeispiel findet also eine Funktionsvorrichtung in Form einer Rauchgasabsaugvorrichtung 14 Verwendung.

Der Chirurgiegenerator 12 und die Rauchgasabsaugvorrichtung 14 sind auf einem Transportwagen 20 abgestellt. Auch der Fußschalter 16 kann auf dem Transportwagen 20 abgestellt werden. Dies ermöglicht die Chirurgievorrichtung 10 zu ihrem Einsatzort, beispielweise einen Körper eines Patienten zu bringen. Die Chirurgievorrichtung 10 kann insbesondere zur Behandlung von Gewebe des Körpers, beispielsweise Schneiden und/oder Koagulieren des Gewebes, verwendet werden.

Der Fußschalter 16 ist über ein Schalterkabel 30 mit dem Chirurgiegenerator 12 verbunden und dient zum Aktivieren und Deaktivieren des Chirurgiegenerators 12. Der Fußschalter 16 kann beispielsweise mit Hilfe von zwei oder einem Fuß betätigt werden, so dass ein Signal über das Schalterkabel 30 zum Aktivieren des Chirurgiegenerators 12 gesendet wird. Wird der Fuß vom Fußschalter 16 genommen, wird der Chirurgiegenerator 12 deaktiviert. Alternativ kann auch eine andere Art von Schalter, beispielsweise ein Handschalter verwendet werden (nicht gezeigt).

Der kapazitive Sensor 18 hat eine Messelektronik 22 und zwei Messelektroden 26a und 26b, die miteinander über das Sensorkabel 24 verbunden sind. In diesem Ausführungsbeispiel enthält die Rauchgasabsaugvorrichtung 14 die Messelektronik 22 des kapazitiven Sensors 18. Die Messelektroden 26a und 26b bilden einen jeweiligen Teil von Folien 28a und 28b, die auf dem Fußschalter 16 aufgeklebt sind. Alternativ können die Messelektroden 26a und 26b auch auf den Folien 28a und 28b aufgeklebt sein. Die Messelektroden 26a und 26b können beispielsweise als eine Metalloxid-Beschichtung auf die Folien 28a und 28b aufgebracht sein. Die Folien 28a und 28b sind in diesem Ausführungsbeispiel Einweg-Folien zur einmaligen Verwendung, die leicht wieder vom Fußschalter 16 entfernbar sind. Dies ermöglicht das Entfernen der Folien 28a und 28b zum Autoklavieren des Fußschalters 16. Nach dem Autoklavieren können dann neue Einweg-Folien 28a und 28b mit Elektroden 26a und 26b auf den Fußschalter 16 aufgeklebt werden. In diesem Ausführungsbeispiel ist auch das Sensorkabel 24 ein Einweg-Kabel, so dass Messelektroden 26a und 26b, Folien 28a und 28b und Sensorkabel 24 vor jeder Verwendung der Chirurgievorrichtung 10 ausgetauscht werden, um sicherzustellen, dass die Chirurgievorrichtung 10 steril ist. Alternativ können die Folien 28a und 28b, Messelektroden 26a und 26b und das Sensorkabel 24 auch wiederverwendbar sein, beispielsweise, wenn eine weniger sterile Umgebung notwendig ist.

Anstatt von Folien kann beispielsweise auch ein Draht oder ein Drahtgeflecht oder können auch mehrere Drähte oder andere kapazitive Messeinrichtungen verwendet werden. Der Draht oder die Drähten können beispielsweise aufgelegt oder festgeklemmt sein. In diesem Ausführungsbeispiel dient die Folie als kapazitive Messeinrichtung.

Der kapazitive Sensor 18 kann eine Kapazität und eine Kapazitätsänderung an den Messelektroden 26a und 26b messen. Der kapazitive Sensor 18 ist ausgebildet in Abhängigkeit von der gemessenen Kapazitätsänderung oder Kapazität die Rauchgasabsaugvorrichtung 14 zu aktivieren oder zu deaktivieren. Wenn ein Fuß auf einer der Messelektroden 26a oder 26b platziert wird, misst die Messelektronik 22 des kapazitiven Sensors 18 eine solche Kapazitätsänderung und Kapazität an der Messelektrode 26a oder 26b, dass sie ein Aktivierungssignal an die Rauchgasabsaugvorrichtung 14 sendet, um diese zu aktivieren. Hierfür ist ein Schwellenwert der Kapazitätsänderung und Kapazität in der Messelektronik 22 definiert. Überschreitet der Messwert der Kapazitätsänderung den Schwellenwert der Kapazitätsänderung, wird das Aktivierungssignal an die Rauchgasabsaugvorrichtung 14 gesendet und diese aktiviert. Alternativ kann die Messelektronik 22 auch derart eingestellt sein, dass bei Überschreiten des Schwellenwertes der Kapazität das Aktivierungssignal an die Rauchgasabsaugvorrichtung 14 gesendet wird, um diese zu aktivieren. Ferner kann die Messelektronik 22 auch derart eingestellt sein, dass das Aktivierungssignal nur an die Rauchgasabsaugvorrichtung 14 gesendet wird, wenn sowohl Schwellenwert der Kapazitätsänderung, als auch Schwellenwert der Kapazität überschritten sind. Wenn der Fuß wieder von der Messelektrode 26a oder 26b entfernt wird und kein Fuß mehr auf einer der Messelektroden 26a oder 26b platziert ist, wird die Rauchgasabsaugvorrichtung 14 von der Messelektronik 22 des kapazitiven Sensors 18 deaktiviert. Hierfür kann ein Deaktivierungssignal an die Rauchgasabsaugvorrichtung 14 gesendet werden, um diese zu deaktivieren. Dadurch, dass die Messelektroden 26a und 26b auf dem Fußschalter 16 aufgeklebt sind, wird sichergestellt, dass beim Betätigen des Fußschalters 16 zum Aktivieren des Chirurgiegenerators 12 auch die Rauchgasabsaugvorrichtung 14 automatisch aktiviert wird und beim Deaktivieren der Rauchgasabsaugvorrichtung 14 auch der Chirurgiegenerator 12 deaktiviert wird. Dies ermöglicht ein nahezu synchrones Aktivieren und Deaktivieren der Rauchgasabsaugvorrichtung 14 und des Chirurgiegenerators 12.

Der kapazitive Sensor 18 ist in diesem Ausführungsbeispiel ein Berührungssensor. Alternativ kann der kapazitive Sensor 18 beispielsweise auch ein Näherungssensor sein. Eine Kapazitätsänderung wird insbesondere dann vom kapazitiven Sensor 18 gemessen, wenn sich ein Objekt, beispielsweise ein Fuß, eine Hand, oder dergleichen der Messelektrode 26a oder 26b des kapazitiven Sensors nähert, um auf dieser platziert zu werden. Das sich nähernde Objekt stellt eine sich gegenüber der Oberfläche der Messelektrode 26a oder 26b bewegende Fläche dar. Zusammen bilden die Messelektrode 26a oder 26b und das sich dieser nähernde Objekt zwei Platten eines Kondensators. Insbesondere durch die Abstandsänderung der Oberfläche des Objektes zur Oberfläche der Messelektrode 26a oder 26b ändert sich die Kapazität des aus der Messelektrode 26a oder 26b und dem Objekt gebildeten Kondensators. Die Messelektronik 22 des kapazitiven Sensors 18 misst die Kapazitätsänderung und/oder Kapazität an der Messelektrode 26a oder 26b und aktiviert oder deaktiviert in Abhängigkeit von dieser die Rauchgasabsaugvorrichtung 14. Somit fungiert der kapazitive Sensor 18 als Schalter für die Rauchgasabsaugvorrichtung 14.

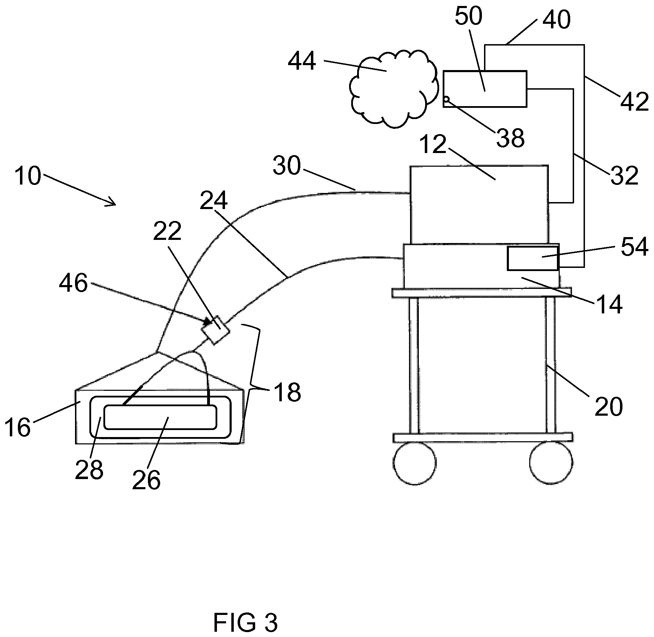

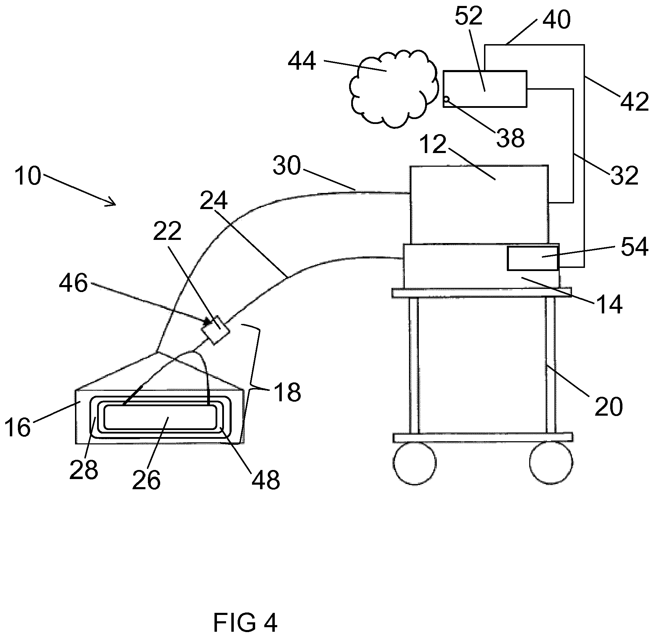

Optional kann der Nutzer der Chirurgievorrichtung 10 auch keinen seiner Füße auf die Messelektroden 26a und 26b platzieren. Beispielsweise indem der Nutzer einen Fuß zwischen die Messelektroden 26a und 26b platziert. Dadurch kann der Nutzer den Fußschalter 16 betätigen, um den Chirurgiegenerator 12 zu aktivieren, ohne dass die Rauchgasabsaugvorrichtung 14 aktiviert wird. Alternativ können die Messelektroden 26a und 26b derart geformt sein, dass sie sich zusammen über die gesamte Fläche des Fußschalters 16 erstrecken. In diesem Fall führt das Betätigen der Fußschalters 16 bei oder unmittelbar vor dem Aktivieren des Chirurgiegenerators 12 zur automatischen Aktivierung der Rauchgasabsaugvorrichtung 14. Es kann auch nur eine Messelektrode 26 verwendet werden, insbesondere eine Messelektrode 26, die sich im Wesentlichen über die gesamte Fläche des Fußschalters 16 erstreckt (vgl. Fig. 3 und Fig. 4).

An den Chirurgiegenerator 12 ist über eine elektrische Leitung 32 in diesem Ausführungsbeispiel ein Elektrochirurgieinstrument 34 angeschlossen. Das Elektrochirurgieinstrument 34 kann ein Teil der Chirurgievorrichtung 10 sein oder mit dieser verbunden sein. Das Elektrochirurgieinstrument 34 hat Elektroden 36, die zum Schneiden und Koagulieren dienen und von dem Chirurgiegenerator 12 mit einer hochfrequenten Wechselspannung versorgt werden können. Das Elektrochirurgieinstrument 34 hat des Weiteren eine Öffnung 38, die mit einem Lumen 40 einer Leitung 42 verbunden ist. Die Leitung 42 ist mit der Rauchgasabsaugvorrichtung 14 verbunden, so dass die Rauchgasabsaugvorrichtung 14 über die Öffnung 38 und das Lumen 40 Rauchgas 44, das beim Schneiden und/oder Koagulieren von Gewebe mit dem Elektrochirurgieinstrument 34 entsteht, absaugen kann.

Der Chirurgiegenerator 12 ist ausgebildet Energie für ein energiebasiertes Chirurgieinstrument, in diesem Ausführungsbeispiel für das Elektrochirurgieinstrument 34, bereitzustellen. Alternativ zu dem Elektrochirurgieinstrument 34 kann auch ein anderes energiebasiertes Chirurgieinstrument an den Chirurgiegenerator 12 angeschlossen sein und von diesem mit Energie versorgt werden. Als alternativ anschließbare energiebasierte Chirurgieinstrumente kommen beispielsweise ein Ultraschallchirurgieinstrument 50 (vgl. Fig. 3) oder ein Laserchirurgieinstrument 52 (vgl. Fig. 4) in Frage.

Die Rauchgasabsaugvorrichtung 14 ist ausgebildet das Rauchgas 44 abzusaugen. Hierfür hat die Rauchgasabsaugvorrichtung 14 eine Rauchgasabsaugpumpe 54, die das Rauchgas 44 über die Öffnung 38 und das Lumen 40 der Leitung 42 in die Rauchgasabsaugvorrichtung 14 einsaugt, um dieses aus der Umgebung, insbesondere von dem zu behandelnden Gewebe zu entfernen. Dies ermöglicht die Sicht auf das zu behandelnde Gewebe zu verbessern.

Die Chirurgievorrichtung 10 stellt keine Anforderungen an die Kompatibilität des Chirurgiegenerators 12, des Elektrochirurgieinstruments 34 oder des Fußschalters 16 zur Rauchgasabsaugvorrichtung 14. Der kapazitive Sensor 18 und die Rauchgasabsaugvorrichtung 14 können daher mit geeigneten bekannten Chirurgievorrichtungen verbunden werden. Dies ermöglicht ein Nachrüsten von geeigneten bekannten Chirurgievorrichtungen.

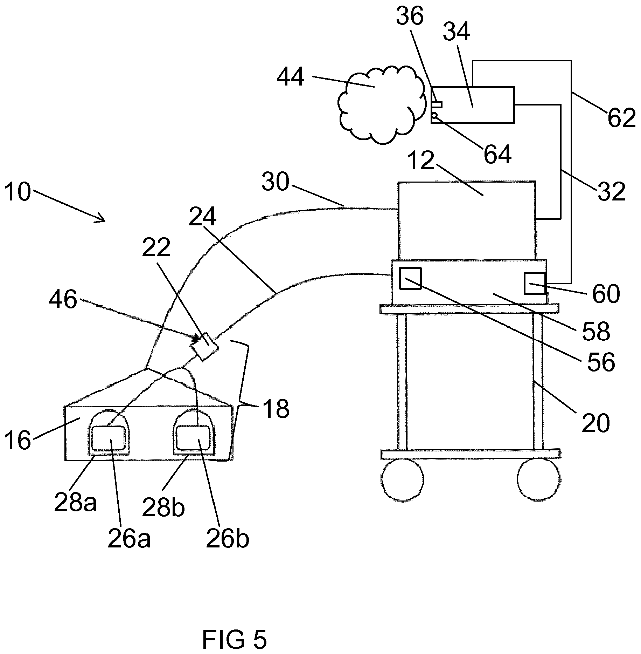

Anstatt der Rauchgasabsaugvorrichtung 14 kann die Chirurgievorrichtung 10 auch eine andere Funktionsvorrichtung oder mehrere Funktionsvorrichtungen aufweisen. Beispielsweise ein Blinklicht 56, eine Beleuchtungsvorrichtung 58 (vgl. Fig. 5), eine Steuerung für ein Ladegerät, eine Videoaufzeichnungsvorrichtung, eine Audioaufzeichnungsvorrichtung, eine Temperaturregulierungsvorrichtung oder beispielsweise auch eine Steuerungseinheit für weitere externe Vorrichtungen, wie zum Beispiel eine Pumpe zur Kühlmittelversorgung oder einen Computer, ein Mobiltelefon, einen Tablet-Computer oder dergleichen.

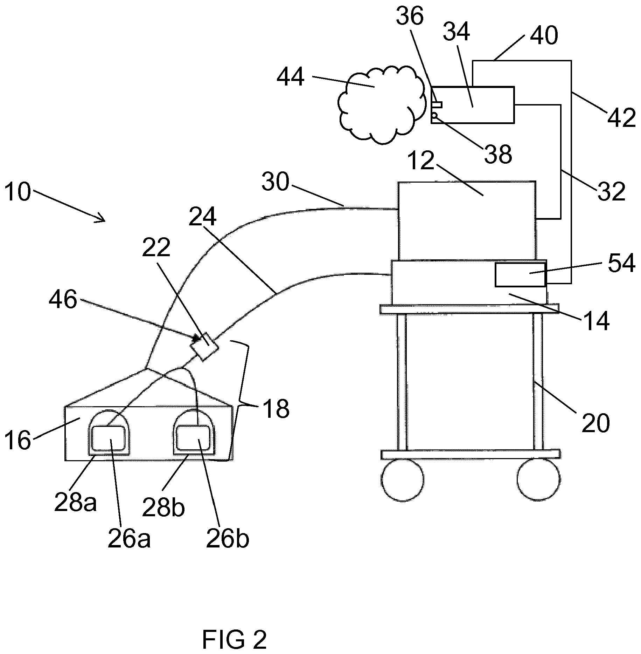

Figur 2 zeigt ein zweites Ausführungsbeispiel der Chirurgievorrichtung 10. Das zweite Ausführungsbeispiel ist im Wesentlichen identisch zu dem ersten Ausführungsbeispiel. Im Gegensatz zum ersten Ausführungsbeispiel ist jedoch beim zweiten Ausführungsbeispiel der Chirurgievorrichtung 10 die Messelektronik 22 nicht in der Rauchgasabsaugvorrichtung 14 angeordnet, sondern in einem Messelektronikgehäuse 46. Das Messelektronikgehäuse 46 ist zwischen den Messelektroden 26a und 26b und der Rauchgasabsaugvorrichtung 14 an dem Sensorkabel 24 angeordnet. Dies ermöglicht es die Messelektronik 22 näher an die Messelektroden 26a und 26b anzuordnen und somit Störeinflüsse zu verringern.

In diesem Ausführungsbeispiel sind die Folien 28a und 28b Einweg-Folien und das Sensorkabel 24 mit dem Messelektronikgehäuse 46 wiederverwendbar. Alternativ können auch die Folien 28a und 28b wiederverwendbar sein oder das Sensorkabel 24 ein Einweg-Kabel, das zur einmaligen Verwendung vorgesehen ist.

Die Funktionsweise des zweiten Ausführungsbeispiels der Chirurgievorrichtung 10 ist im Wesentlichen gleich zur Funktionsweise des ersten Ausführungsbeispiels. Auch im zweiten Ausführungsbeispiel der Chirurgievorrichtung 10 hat die Chirurgievorrichtung 10 eine Funktionsvorrichtung in Form einer Rauchgasabsaugvorrichtung 14.

Figur 3 zeigt ein drittes Ausführungsbeispiel der Chirurgievorrichtung 10. Das dritte Ausführungsbeispiel ist im Wesentlichen identisch zu dem zweiten Ausführungsbeispiel. Im Gegensatz zum zweiten Ausführungsbeispiel ist jedoch beim dritten Ausführungsbeispiel ein Ultraschallchirurgieinstrument 50 an den Chirurgiegenerator 12 angeschlossen. Dieses verwendet Ultraschallwellen zum Schneiden von Gewebe. Ferner ist auf dem Fußschalter 16 eine einzelne Messelektrode 26 und Folie 28 aufgeklebt anstatt zweier Messelektroden 26a und 26b. Die Messelektrode 26 nimmt fast die gesamte Fläche des Fußschalters 16 ein.

Die Funktionsweise des dritten Ausführungsbeispiels der Chirurgievorrichtung 10 ist im Wesentlichen gleich zur Funktionsweise des ersten Ausführungsbeispiels. Auch im dritten Ausführungsbeispiel der Chirurgievorrichtung 10 hat die Chirurgievorrichtung 10 eine Funktionsvorrichtung in Form einer Rauchgasabsaugvorrichtung 14.

Figur 4 zeigt ein viertes Ausführungsbeispiel der Chirurgievorrichtung 10. Das vierte Ausführungsbeispiel ist im Wesentlichen identisch zu dem dritten Ausführungsbeispiel. Im Gegensatz zum dritten Ausführungsbeispiel ist jedoch beim vierten Ausführungsbeispiel ein Laserchirurgieinstrument 52 an den Chirurgiegenerator 12 angeschlossen. Dieses verwendet Laserstrahlen zum Schneiden und/oder Koagulieren von Gewebe. Ferner ist auf der Folie 28 um die Messelektrode 26 herum eine Schirmelektrode 48 angeordnet. Die Schirmelektrode 48 dient dazu einen inhomogenen Randbereich des elektrischen Feldes von der Messelektrode 26 abzuschirmen.

Auch die Funktionsweise des vierten Ausführungsbeispiels der Chirurgievorrichtung 10 ist im Wesentlichen gleich zur Funktionsweise des ersten Ausführungsbeispiels. Auch im vierten Ausführungsbeispiel der Chirurgievorrichtung 10 hat die Chirurgievorrichtung 10 eine Funktionsvorrichtung in Form einer Rauchgasabsaugvorrichtung 14.

Figur 5 zeigt ein fünftes Ausführungsbeispiel der Chirurgievorrichtung 10. Das fünfte Ausführungsbeispiel ist im Wesentlichen identisch zu dem zweiten Ausführungsbeispiel. Im Gegensatz zum zweiten Ausführungsbeispiel weist jedoch beim fünften Ausführungsbeispiel die Chirurgievorrichtung 10 anstatt einer Rauchgasabsaugvorrichtung 14 ein Blinklicht 56 und eine Beleuchtungsvorrichtung 58 auf. Diese sind mit der Messelektronik 22 des kapazitiven Sensors 18 verbunden. In diesem Fall ist die Funktionsvorrichtung also eine Beleuchtungsvorrichtung 58, die zusätzlich das Blinklicht 56 aufweist. Der kapazitive Sensor 18 aktiviert in diesem Ausführungsbeispiel zuerst das Blinklicht 56 und nachfolgend die Beleuchtungsvorrichtung 58. Alternativ können diese beispielsweise auch synchron aktiviert werden.

Das Blinklicht 56 dient dazu den Nutzer der Chirurgievorrichtung 10 zu warnen, wenn die Beleuchtungsvorrichtung 58 aktiviert wird. Ferner dient es zugleich als Warnung für das Aktivieren des Chirurgiegenerators 12 und somit für die Aktivierung des Elektrochirurgieinstruments 34. Wenn eine synchrone Aktivierung des Blinklichts 56 und der Beleuchtungsvorrichtung 58 erfolgt, dient das Blinklicht 56 als Status-Anzeige für den aktivierten Zustand der Beleuchtungsvorrichtung 58.

Die Beleuchtungsvorrichtung 58 hat eine Lichtquelle 60 und ist über die Leitung 62 mit einer Austrittsöffnung 64 des Elektrochirurgieinstruments 34 verbunden. Die Lichtquelle 60 der Beleuchtungsvorrichtung 58 stellt ein spezielles Licht bereit, das über die Leitung 62 der Austrittsöffnung 64 zugeführt wird, um das zu behandelnde Gewebe zu beleuchten, wenn das Elektrochirurgieinstrument 34 verwendet wird. Dies ermöglicht es die Sicht auf das zu behandelnde Gewebe zu verbessern. Alternativ kann eine Lichtquelle für die Bereitstellung des speziellen Lichts auch im Elektrochirurgieinstrument 34 angeordnet sein und die Beleuchtungsvorrichtung 58 dazu dienen die Lichtquelle mit Energie zu versorgen.

Die Funktionsweise des fünften Ausführungsbeispiels der Chirurgievorrichtung 10 ist ähnlich zur Funktionsweise des ersten Ausführungsbeispiels und unterscheidet sich im Wesentlichen dadurch, dass die Funktionsvorrichtung in diesem Fall keine Rauchgasabsaugvorrichtung 14 ist, sondern eine Beleuchtungsvorrichtung 58 mit einem Blinklicht 56.

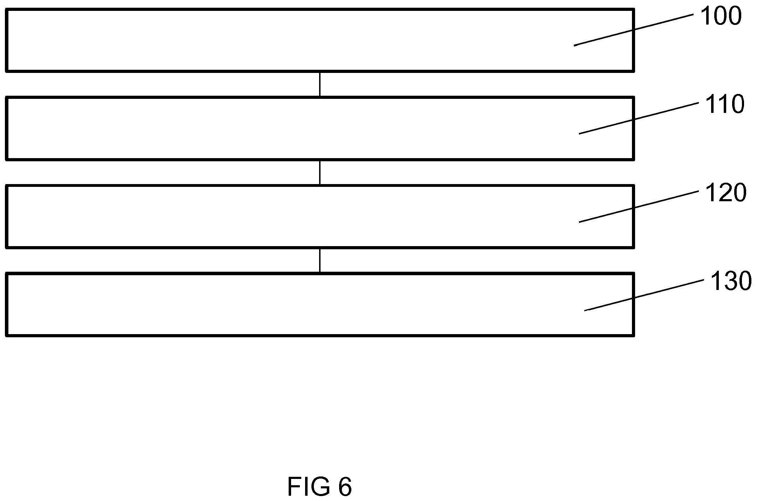

Figur 6 zeigt ein Blockdiagramm eines Ausführungsbeispiels eines Verfahrens zum im Wesentlichen synchronen automatischen Aktivieren und Deaktivieren einer Funktionsvorrichtung in Form einer Rauchgasabsaugvorrichtung und eines Chirurgiegenerators einer Chirurgievorrichtung. Die Chirurgievorrichtung weist einen Schalter zum Aktivieren und Deaktivieren des Chirurgiegenerators und eine an dem Schalter angeordnete Messelektrode eines kapazitiven Sensors zum Aktivieren und Deaktivieren der Rauchgasabsaugvorrichtung auf. Das Verfahren umfasst die folgenden Schritte:
100 Platzieren eines Objektes auf der Messelektrode des kapazitiven Sensors.
110 Messen einer Kapazitätsänderung an der Messelektrode durch den kapazitiven Sensor. Alternativ oder zusätzlich kann auch eine Kapazität an der Messelektrode gemessen werden.
120 automatisches Aktivieren oder automatisches Deaktivieren der Rauchgasabsaugvorrichtung, wenn ein vorbestimmter Schwellenwert einer Kapazitätsänderung überschritten wird. Alternativ oder zusätzlich kann die Rauchgasabsaugvorrichtung auch automatisch aktiviert oder automatisch deaktiviert werden, wenn ein vorbestimmter Schwellenwert einer Kapazität überschritten wird oder unterschritten wird. Wenn Kapazitätsänderung und Kapazität gemessen werden und für beide ein vorbestimmter Schwellenwert vorliegt, kann die automatische Aktivierung und automatische Deaktivierung der Rauchgasabsaugvorrichtung auch vom überschreiten oder unterschreiten beider Schwellenwerte abhängen.
130 Betätigen des Schalters zum Aktivieren des Chirurgiegenerators oder Entfernen des Objektes vom Schalter zum Deaktivieren des Chirurgiegenerators.

In diesem Ausführungsbeispiel des Verfahrens ist der Schwellenwert der Kapazitätsänderung derart gewählt, dass die Rauchgasabsaugvorrichtung automatisch aktiviert wird, wenn der Schalter betätigt wird und automatisch deaktiviert wird, wenn das Objekt vom kapazitiven Sensor entfernt wird. Hierdurch wird sichergestellt, dass ein im Wesentlichen synchrones automatisches Aktivieren und Deaktivieren des Chirurgiegenerators und der Rauchgasabsaugvorrichtung erfolgt. Alternativ oder zusätzlich kann der Schwellenwert der Kapazität derart gewählt werden, dass die Rauchgasabsaugvorrichtung aktiviert wird, wenn der Schalter betätigt wird und deaktiviert wird, wenn das Objekt vom kapazitiven Sensor entfernt wird.

Des Weiteren kann alternativ der Schwellenwert derart eingestellt werden, dass die Rauchgasabsaugvorrichtung bereits vor dem Betätigen des Schalters aktiviert wird, beispielsweise, wenn ein Objekt nahe an die Messelektrode des kapazitiven Sensors gebracht wird. Dies ermöglicht ein Aktivieren der Rauchgasabsaugvorrichtung ohne, dass der Chirurgiegenerator aktiviert wird.

Das Verfahren kann alternativ auch mit einer anderen oder mehreren anderen Funktionsvorrichtungen ausgeführt werden. Beispielsweise kann die Funktionsvorrichtung in Form eines Blinklichts, einer Beleuchtungsvorrichtung, einer Steuerung für ein Ladegerät, einer Videoaufzeichnungsvorrichtung, einer Audioaufzeichnungsvorrichtung, einer Temperaturregulierungsvorrichtung oder beispielsweise auch einer Steuerungseinheit für weitere externe Vorrichtungen, wie zum Beispiel eine Pumpe zur Kühlmittelversorgung oder einen Computer, ein Mobiltelefon, einen Tablet-Computer oder dergleichen vorliegen.

### Bezugszeichenliste

- 10: Chirurgievorrichtung
- 12: Chirurgiegenerator
- 14: Rauchgasabsaugvorrichtung
- 16: Fußschalter
- 18: kapazitiver Sensor
- 20: Transportwagen
- 22: Messelektronik
- 24: Sensorkabel
- 26, 26a, 26b: Messelektrode
- 28, 28a, 28b: Folie
- 30: Schalterkabel
- 32: elektrische Leitung
- 34: Elektrochirurgieinstrument
- 36: Elektroden
- 38: Öffnung
- 40: Lumen
- 42: Leitung
- 44: Rauchgas
- 46: Messelektronikgehäuse
- 48: Schirmelektrode
- 50: Ultraschallchirurgieinstrument
- 52: Laserchirurgieinstrument
- 54: Rauchgasabsaugpumpe
- 56: Blinklicht
- 58: Beleuchtungsvorrichtung
- 60: Lichtquelle
- 62: Leitung
- 64: Austrittsöffnung

## Patentansprüche

1. Chirurgievorrichtung (10) zum im Wesentlichen synchronen automatischen Aktivieren und Deaktivieren einer Funktionsvorrichtung (14, 56, 58) und eines Chirurgiegenerators (12) der Chirurgievorrichtung (10), umfassend
- den Chirurgiegenerator (12), der ausgebildet ist Energie für ein energiebasiertes Chirurgieinstrument (34, 50, 52) bereitzustellen,
- die Funktionsvorrichtung (14, 56, 58), die ausgebildet ist in einem aktivierten Zustand eine Funktion bereitzustellen,
- einen Schalter (16) zum Aktivieren und Deaktivieren des Chirurgiegenerators (12),
- einen kapazitiven Sensor (18), der wenigstens eine Messelektrode (26, 26a, 26b) aufweist, die an dem Schalter (16) angeordnet ist,
wobei der kapazitive Sensor (18) ausgebildet ist eine Kapazitätsänderung oder Kapazität an der Messelektrode (26, 26a, 26b) zu messen und in Abhängigkeit von der gemessenen Kapazitätsänderung oder Kapazität die Funktionsvorrichtung (14, 56, 58) zu aktivieren oder zu deaktivieren.

2. Chirurgievorrichtung (10) gemäß Anspruch 1, wobei die Messelektrode (26, 26a, 26b) derart an dem Schalter (16) angeordnet ist und der kapazitive Sensor (18) derart eingestellt ist, dass ein Aktivieren des Chirurgiegenerators (12) auch ein Aktivieren der Funktionsvorrichtung (14, 56, 58) bewirkt.

3. Chirurgievorrichtung (10) gemäß Anspruch 1 oder 2, wobei die Messelektrode (26, 26a, 26b) derart an dem Schalter (16) angeordnet ist und der kapazitive Sensor (18) derart eingestellt ist, dass ein Deaktivieren der Funktionsvorrichtung (14, 56, 58) auch ein Deaktivieren des Chirurgiegenerators (12) bewirkt.

4. Chirurgievorrichtung (10) gemäß einem der Ansprüche 1 bis 3, wobei der kapazitive Sensor (18) derart ausgebildet ist, dass ein Aktivieren oder Deaktivieren der Funktionsvorrichtung (14, 56, 58) im Wesentlichen synchron zum Aktivieren oder Deaktivieren des Chirurgiegenerators (12) erfolgt.

5. Chirurgievorrichtung (10) gemäß einem der Ansprüche 1 bis 4, das eine auf dem Schalter (16) befestigbare kapazitive Messeinrichtung (28, 28a, 28b) aufweist und wobei die kapazitive Messeinrichtung (28, 28a, 28b) die Messelektrode (26, 26a, 26b) des kapazitiven Sensors (18) aufweist.

6. Chirurgievorrichtung (10) gemäß einem der Ansprüche 1 bis 5, wobei der kapazitive Sensor (18) eine Messelektronik (22) aufweist, die ausgebildet ist die Kapazitätsänderung oder die Kapazität an der Messelektrode (26, 26a, 26b) zu messen.

7. Chirurgievorrichtung (10) gemäß Anspruch 6, wobei die Messelektronik (22) des kapazitiven Sensors (18) in der Funktionsvorrichtung (14, 56, 58) angeordnet ist.

8. Chirurgievorrichtung (10) gemäß Anspruch 6, wobei der kapazitive Sensor (18) über ein Kabel (24) mit der Funktionsvorrichtung (14, 56, 58) verbunden ist und die Messelektronik (22) des kapazitiven Sensors (18) an dem Kabel (24) zwischen Messelektrode (26, 26a, 26b) und Funktionsvorrichtung (14, 56, 58) angeordnet ist.

9. Chirurgievorrichtung (10) gemäß einem der Ansprüche 1 bis 8, wobei der kapazitive Sensor (18) wenigstens eine Schirmelektrode (48) aufweist, die nahe der Messelektrode (26, 26a, 26b) angeordnet ist und dazu ausgebildet ist einen inhomogenen Randbereich des elektrischen Feldes von der Messelektrode (26, 26a, 26b) abzuschirmen.

10. Chirurgievorrichtung (10) gemäß einem der Ansprüche 1 bis 9, wobei der Schalter (16) ein Fußschalter (16) ist, der ausgebildet ist mit Hilfe eines Fußes oder mehrerer Füße betätigt zu werden.

11. Chirurgievorrichtung (10) gemäß einem der Ansprüche 1 bis 10, das ein energiebasiertes Chirurgieinstrument (34, 50, 52) aufweist, wobei das energiebasierte Chirurgieinstrument (34, 50, 52) dazu ausgebildet ist Gewebe zu schneiden und/oder zu koagulieren und wobei das energiebasierte Chirurgieinstrument (34, 50, 52)
- ein Ultraschallchirurgieinstrument (50),
- ein Laserchirurgieinstrument (52), oder
- ein Elektrochirurgieinstrument (34) ist, wobei das Elektrochirurgieinstrument (34) wenigstens eine Elektrode (36) aufweist und dazu ausgebildet ist mittels einer hochfrequenten Wechselspannung betrieben zu werden, um Gewebe zu schneiden und/oder zu koagulieren.

12. Verfahren zum automatischen Aktivieren und Deaktivieren einer Funktionsvorrichtung (14, 56, 58) einer Chirurgievorrichtung (10),
wobei die Chirurgievorrichtung (10) einen Schalter (16) zum Aktivieren und Deaktivieren eines Chirurgiegenerators (12) und eine an dem Schalter (16) angeordnete Messelektrode (26, 26a, 26b) eines kapazitiven Sensors (18) zum Aktivieren und Deaktivieren der Funktionsvorrichtung (14, 56, 58) aufweist und
wobei das Verfahren die folgenden Schritte umfasst:
- Platzieren eines Objektes auf der Messelektrode (26, 26a, 26b) des kapazitiven Sensors (18),
- Messen einer Kapazität oder Kapazitätsänderung an der Messelektrode (26, 26a, 26b) durch den kapazitiven Sensor (18),
- automatisches Aktivieren oder automatisches Deaktivieren der Funktionsvorrichtung (14, 56, 58), wenn ein vorbestimmter Schwellenwert einer Kapazitätsänderung oder Kapazität an der Messelektrode (26, 26a, 26b) überschritten wird, wobei das Aktivieren der Funktionsvorrichtung kurz vor dem Aktivieren des Chirurgiegenerators erfolgt.

## Claims

1. A surgical device (10) for essentially synchronous automatic activating and deactivating of a functional device (14, 56, 58) and a surgical generator (12) of a surgical device (10), comprising:
- the surgical generator (12) configured to provide energy for an energy based surgical instrument (34, 50, 52);
- the functional device (14, 56, 58) configured to provide a function in an activated condition;
- a switch (16) for activating and deactivating the surgical generator (12)
- a capacitive sensor (18) including at least one measuring electrode (26, 26a, 26b) that is arranged at the switch (16),
wherein the capacitive sensor (18) is configured to measure a capacity change or a capacity at the measuring electrode (26, 26a, 26b) and activate or deactivate the functional device (14, 56, 58) as a function of a measured capacity change or capacity.

2. The surgical device (10) according to claim 1, wherein the measuring electrode (26, 26a, 26b) is arranged at the switch (16) and the capacitive sensor (18) is adjusted so that activating the surgical generator (12) also causes activating the functional device (14, 56, 58).

3. The surgical device (10) according to claim 1 or 2, wherein the measuring electrode (26, 26a, 26b) is arranged at the switch (16) and the capacitive sensor (18) is adjusted so that deactivating the functional device (14, 56, 58) also causes deactivating the surgical generator (12).

4. The surgical device (10) according to one of the claims 1 through 3, wherein the capacitive sensor (18) is configured so that activating or deactivating the functional device (14, 56, 58) is performed essentially synchronously with activating or deactivating the surgical generator (12).

5. The surgical device (10) according to one of the claims 1 through 4, further comprising a capacitive measuring device (28, 28a, 28b) that is attachable at the switch (16), wherein the capacitive measuring device (28, 28a, 28b) includes the measuring electrode (26, 26a, 26b) of the capacitive sensor (18).

6. The surgical device (10) according to one of the claims 1 through 5, wherein the capacitive sensor (18) includes measuring electronics (22) configured to measure the capacity change or the capacity at the measuring electrode (26, 26a, 26b).

7. The surgical device (10) according to claim 6, wherein measuring electronics (22) of the capacitive sensor (18) are arranged in the functional device (14, 56, 58).

8. The surgical device (10) according to claim 6, wherein the capacitive sensor (18) is connected with the functional device (14, 56, 58) through a cable (24) and the measuring electronics (22) of the capacitive sensor (18) are arranged at the cable (24) between the measuring electrode (26, 26a, 26b) and the functional device (14, 56, 58).

9. The surgical device (10) according to one of the claims 1 through 8 wherein the capacitive sensor (18) includes at least one shielding electrode (48) which is arranged proximal to the measuring electrode (26, 26a, 26b) and configured to shield in an non-homogenous edge portion of an electrical field of the measuring electrode (26, 26a, 26b).

10. The surgical device (10) according to one of the claims 1 through 9, wherein the switch (16) is a foot switch (16) that is configured to be actuated by a foot or by plural feet.

11. The surgical device (10) according to one of the claims 1 - 10, further comprising and energy based surgical instrument (34, 50, 52) wherein the energy based surgical instrument (34, 50, 52) is configured to cut and/or coagulate tissue and wherein the energy based surgical instrument (34, 50, 52) is
- an ultrasound surgical instrument (50)
- a laser surgical instrument (52) or
- an electrosurgical instrument (34), wherein the electro surgical instrument (34) includes at least one electrode (36) and is configured to be operated by a high frequency alternating voltage to cut, and/or coagulate tissue.

12. A method for automatic activating and deactivating of a functional device (14, 56, 58) of a surgical device (10), wherein the surgical device (10) includes a switch (16) for activating and deactivating a surgical generator (12) and a measuring electrode (26, 26a, 26b) of a capacitive sensor (18) arranged at the switch (16) for activating and deactivating the functional device (15, 56, 58), the method comprising the following steps:
- placing an object onto the measuring electrode (26, 26a, 26b) of the capacitive sensor (18).
- measuring a capacity or capacity change at the measuring electrode (26, 26a, 26b) through the capacitive sensor (18),
- automatic activating or deactivating of a functional device (14, 56, 58), when a predetermined threshold value of a capacity change or a capacity is exceeded at the measuring electrode (26, 26a, 26b), wherein the activating of the functional device occurs shortly before the activating of the surgical generator.

## Revendications

1. Dispositif chirurgical (10) permettant l'activation et la désactivation automatique, de manière essentiellement synchrone, d'un dispositif fonctionnel (14, 56, 58) et d'un générateur chirurgical (12) du dispositif chirurgical (10), comprenant
- le générateur chirurgical (12), qui est conçu pour fournir de l'énergie à un instrument chirurgical (34, 50, 52) basé sur l'énergie,
- le dispositif fonctionnel (14, 56, 58), qui est conçu pour fournir une fonction dans un état activé,
- un commutateur (16) permettant l'activation et la désactivation du générateur chirurgical (12),
- un capteur capacitif (18) qui comporte au moins une électrode de mesure (26, 26a, 26b) qui est disposée sur le commutateur (16),
le capteur capacitif (18) étant conçu pour mesurer une variation de capacité ou une capacité sur l'électrode de mesure (26, 26a, 26b) et pour activer ou désactiver le dispositif fonctionnel (14, 56, 58) en fonction de la variation de capacité ou de la capacité mesurée.

2. Dispositif chirurgical (10) selon la revendication 1, l'électrode de mesure (26, 26a, 26b) étant disposée sur le commutateur (16) et le capteur capacitif (18) étant réglé de telle sorte qu'une activation du générateur chirurgical (12) provoque également une activation du dispositif fonctionnel (14, 56, 58).

3. Dispositif chirurgical (10) selon la revendication 1 ou 2, l'électrode de mesure (26, 26a, 26b) étant disposée sur le commutateur (16) et le capteur capacitif (18) étant réglé de telle sorte qu'une désactivation du dispositif fonctionnel (14, 56, 58) provoque également une désactivation du générateur chirurgical (12).

4. Dispositif chirurgical (10) selon l'une des revendications 1 à 3, le capteur capacitif (18) étant conçu de telle sorte qu'une activation ou une désactivation du dispositif fonctionnel (14, 56, 58) s'effectue essentiellement de manière synchrone avec l'activation ou la désactivation du générateur chirurgical (12).

5. Dispositif chirurgical (10) selon l'une des revendications 1 à 4, qui comporte un équipement de mesure capacitif (28, 28a, 28b) pouvant être fixé sur le commutateur (16) et l'équipement de mesure capacitif (28, 28a, 28b) comportant l'électrode de mesure (26, 26a, 26b) du capteur capacitif (18).

6. Dispositif chirurgical (10) selon l'une des revendications 1 à 5, le capteur capacitif (18) comportant une électronique de mesure (22) qui est conçue pour mesurer la variation de capacité ou la capacité sur l'électrode de mesure (26, 26a, 26b).

7. Dispositif chirurgical (10) selon la revendication 6, l'électronique de mesure (22) du capteur capacitif (18) étant disposée dans le dispositif fonctionnel (14, 56, 58).

8. Dispositif chirurgical (10) selon la revendication 6, le capteur capacitif (18) étant relié au dispositif fonctionnel (14, 56, 58) par un câble (24) et l'électronique de mesure (22) du capteur capacitif (18) étant disposée sur le câble (24) entre l'électrode de mesure (26, 26a, 26b) et le dispositif fonctionnel (14, 56, 58).

9. Dispositif chirurgical (10) selon l'une des revendications 1 à 8, le capteur capacitif (18) comportant au moins une électrode écran (48) disposée à proximité de l'électrode de mesure (26, 26a, 26b) et conçue pour faire écran à une zone périphérique inhomogène du champ électrique provenant de l'électrode de mesure (26, 26a, 26b).

10. Dispositif chirurgical (10) selon l'une des revendications 1 à 9, le commutateur (16) étant un commutateur à pied (16) conçu pour être actionné à l'aide d'un ou de plusieurs pieds.

11. Dispositif chirurgical (10) selon l'une des revendications 1 à 10, qui comporte un instrument chirurgical à base d'énergie (34, 50, 52), l'instrument chirurgical à base d'énergie (34, 50, 52) étant conçu pour couper et/ou coaguler des tissus, et l'instrument chirurgical à base d'énergie (34, 50, 52) étant
- un instrument chirurgical à ultrasons (50),
- un instrument chirurgical au laser (52), ou
- un instrument électro-chirurgical (34), l'instrument électro-chirurgical (34) comportant au moins une électrode (36) et étant conçu pour être alimenté par une tension alternative à haute fréquence afin de couper et/ou de coaguler des tissus.

12. Procédé permettant l'activation et la désactivation automatiques d'un dispositif fonctionnel (14, 56, 58) d'un dispositif chirurgical (10),
le dispositif chirurgical (10) comportant un commutateur (16) permettant l'activation et la désactivation d'un générateur chirurgical (12) et d'une électrode de mesure (26, 26a, 26b) disposée sur le commutateur (16) permettant l'activation et la désactivation du dispositif fonctionnel (14, 56, 58) et le procédé comprenant les étapes suivantes :
- Placement d'un objet sur l'électrode de mesure (26, 26a, 26b) du capteur capacitif (18),
- Mesure d'une capacité ou d'une variation de capacité sur l'électrode de mesure (26, 26a, 26b) par le capteur capacitif (18),
- Activation automatique ou désactivation automatique du dispositif fonctionnel (14, 56, 58) lorsqu'une valeur de seuil prédéterminée d'une variation de capacité ou d'une capacité est dépassée sur l'électrode de mesure (26, 26a, 26b), l'activation du dispositif fonctionnel ayant lieu juste avant l'activation du générateur chirurgical.
